(19) Europäisches Patentamt
European Patent Office
Office européen des brevets

(11) Veröffentlichungsnummer: **0 433 581 A2**

# (12) EUROPÄISCHE PATENTANMELDUNG

(21) Anmeldenummer: 90119047.0

(22) Anmeldetag: 04.10.90

(51) Int. Cl.5: **A61B 17/34**

(30) Priorität: 18.12.89 DE 8914955 U

(43) Veröffentlichungstag der Anmeldung:
**26.06.91 Patentblatt 91/26**

(84) Benannte Vertragsstaaten:
**CH DE FR GB LI NL**

(71) Anmelder: **WISAP GESELLSCHAFT FÜR WISSENSCHAFTLICHEN APPARATEBAU MBH**
**Rudolf-Diesel-Ring 20**
**W-8029 Sauerlach(DE)**

(72) Erfinder: **Semm, Horst**
**Melchiorstrasse 35**
**W-8000 München 71(DE)**

(74) Vertreter: **Heim, Hans-Karl, Dipl.-Ing. et al**
**c/o Weber & Heim Hofbrunnstrasse 36**
**W-8000 München 71(DE)**

(54) **Trokarhülse.**

(57) Eine Trokarhülse mit einem proximalen Hauptkörper axialer, durchgehender Öffnung und mit einer mit dem Hauptkörper verbundenen distalen Hülse ermöglicht ein funktionssicheres und leicht handhabbares Arbeiten, wenn die Hülse lösbar, insbesondere manuell auswechselbar mit dem Hauptkörper verbunden ist.

## Fig. 1

# TROKARHÜLSE

Die Erfindung betrifft eine Trokarhülse gemäß Oberbegriff des Anspruchs 1.

Trokarhülsen dieser Art sind in der endoskopischen Chirurgie allgemein bekannt. Der Aufbau dieser Trokarhülsen ist dabei im wesentlichen einstückig, wobei am oder im proximalen Hauptkörper eine zylindrische Hülse eingelötet oder eingeklebt ist.

Der Hauptkörper, der den fertigungstechnisch komplizierten und daher auch kostenintensiven Teil der Trokarhülse darstellt, weist in der Regel Anschlüsse für chirurgische oder optische Geräte und Gaszuführungen auf. Auch ein manuell einstellbares oder automatisch schließendes Ventil zur Verhinderung eines Gasaustausches zwischen dem Körperinneren und der Außenatmosphäre ist üblicherweise vorgesehen.

Diese Trokarhülsen werden von einem an seiner Spitze konifizierten Trokar durchsetzt und dann z.B. mittels Durchstoßen der Bauchdecke in das Innere des Körpers eingeführt. Die Spitze der Trokarhülse sollte abgeschrägt sein, damit man beim Durchstoßen der Bauchdecke einen geringeren Perforationswiderstand erhält. Nach dem Einführen des distalen Teils der Trokarhülse in das Körperinnere wird der Trokar aus der Trokarhülse herausgezogen, so daß diese eine verschließbare Öffnung zum Körperinneren darstellt, durch die optische oder chirurgische Instrumente in das Körperinnere eingeführt werden können. Oft sind Trokarhülsen auch mit einem Anschluß für eine Gaszufuhr, insbesondere für $CO_2$ versehen, um die Bauchdecke durch Erzeugen eines Überdrucks im Körperinneren anzuheben und somit eine freie Zugänglichkeit zu den inneren Organen zu erhalten.

Bei in der Abdominal-Chirurgie durchgeführten operativen Eingriffen werden chirurgische Instrumente, wie Zangen, Schlingen und Scheren durch die Trokarhülse in das Körperinnere eingeführt. Die Betätigungsglieder dieser Instrumente sind in Ruhestellung axial ausgerichtet, damit sie ohne Probleme durch die Trokarhülse eingeführt werden können. Beim Herausziehen von operativ entfernten Gewebestücken z.B. mit der Biopsie-Zange oder Krallen-Zange ist es üblich, die Zange mit dem gefaßten Gewebe im nicht ganz geschlossenen Zustand mehr oder minder mit Kraft in die Hülse hineinzuziehen, so daß die Zange auf den Durchmesser zwangsweise am distalen Rand der Hülse geschlossen wird, der es erlaubt, das Zangenmaul mit gefaßtem Gewebe in die Trokarhülse einzuziehen. Das distale Ende der Hülse wird dadurch aufgedehnt bzw. aufgeweitet und überragt dann den zylindrischen Trokar in radialer Richtung, wodurch eine traumatische Wirkung hervorgerufen werden kann, da dieser aufgeweitete Bereich messerartig die Gewebeschichten durchstechen kann. Andererseits kann der abgeschrägte, distale Rand der Hülse durch den Kontakt mit den Betätigungsgliedern auch entschärft werden, wobei dies gerade für ein späteres atraumatisches Eingleiten durch das Gewebe, besonders bei einem Z-Stich, sehr nachteilig ist.

Die Gefahr bei den bisher normalerweise mit Weichlot eingelöteten Hülsen in den Hauptkörper war, daß nach mehrfachem Gebrauch und mehrfacher Sterilisierung die Lötstellen brüchig wurden und die gesamte Hülse an der Lötstelle abbrach. Da bei den bisherigen Trokarhülsen das proximale Ende der Hülse nahezu streng zylindrisch war, bestand zudem die Gefahr, daß bei beschädigter Lötstelle die Hülse möglicherweise in das Körperinnere eingleiten konnte.

Um diese Risiken zu vermeiden, mußte die einstückige Trokarhülse, also Hauptkörper und Hülse, an den Hersteller zurückgesandt werden, damit die beschädigte Hülse vom Hauptkörper losgelötet und gegen eine neue Hülse ausgetauscht werden konnte. Da bereits eine undichte Lötstelle zwischen Hülse und Hauptkörper während der Operation zu einem Druckabfall im Körperinneren führen kann und mögliche Komplikationen bereits von vornherein eliminiert werden müssen, bedurfte es daher einer vergleichsweisen großen Anzahl von Reserve-Trokarhülsen. Hinzu kam, daß man auch im Hinblick auf unterschiedliche Längen der Trokarhülsen flexibel sein mußte. Zudem stellte das Aus- und Einlöten der Hülsen einen erheblichen Kostenfaktor dar.

Es ist daher Aufgabe der Erfindung, eine Trokarhülse der gattungsgemäßen Art so zu gestalten, daß diese einen axial ausgerichteten sicheren Sitz der Hülse am Hauptkörper gewährleistet und ein einfaches, kostengünstiges Auswechseln der Hülse möglich ist.

Diese Aufgabe wird bei einer Trokarhülse der gattungsgemässen Art durch die Merkmale des Kennzeichens des Anspruches 1 gelöst.

Durch die erfindungsgemäße Idee kann nunmehr die Hülse bei einer Beschädigung beispielsweise manuell durch simples Aufdrehen der Überwurfmutter gelöst und durch eine neue ersetzt werden. Dieser einfache Auswechselvorgang besteht selbstverständlich auch in Situationen, in denen der Operateur feststellt, daß er eine längere oder kürzere Hülse für die komplette Trokarhülse benötigt, um den operativen Eingriff mit größtmöglicher Sicherheit durchführen zu können.

Selbstverständlich gestattet diese einfache Verbindung zwischen dem Hauptkörper und der Hülse auch bessere Sterilisationsmöglichkeiten. Die Idee eröffnet sogar die Möglichkeit, Hülsen mit unterschiedlichen Durchmessern verwenden zu können, sofern sichergestellt ist, daß über die proximale Bördelung der Hülse eine zuverlässige, dichte Verbindung, nach Möglichkeit selbstzentrierend, mit dem Hauptkörper geschaffen wird. Die axiale Ausrichtung der Hülse mit der Öffnung im Hauptkörper muß dabei stets gewährleistet sein.

In einer vorteilhaften Weiterbildung der Erfindung ist die Hülse an ihrem proximalen Ende aufgebördelt und von einer die Bördelung hintergreifenden Überwurfmutter umfaßt, die form- und/oder kraftschlüssig an dem Hauptkörper festlegbar ist. Durch diese Verbindung wird nicht nur eine schnelle Auswechselbarkeit der Hülse erzielt, sondern auch ein sehr sicherer Sitz der Hülse an dem Hauptkörper. Die Hülse ist durch die Bördelung und die die Bördelung hintergreifende Überwurfmutter auch gegen ein Hineinrutschen in den Körper gesichert.

Durch die Erfindung ist es weiterhin möglich, einen Hauptkörper mit verschieden langen Hülsen zu verbinden, was zu einer Verringerung der Aufwendungen für das chirurgische Instrumentarium beiträgt.

Die Verbindung zwischen Hülse und Überwurfmutter wird bevorzugt mittels eines Gewindes gelöst. Es können aber auch andere Befestigungen als Steck- oder Bajonettverschluß realisiert werden.

In einer vorteilhaften Weiterbildung der Erfindung hat die Überwurfmutter zwei axial hintereinander angeordnete Umfangsbereiche, von denen einer als Anschlußbereich mit dem Hauptkörper und der andere als Griffbereich ausgebildet ist. Ein manueller Hülsenwechsel ist daher sehr schnell durchführbar.

Da bei einem abdominalen chirurgischen Eingriff der Innenraum des Körpers gegenüber der Atmosphäre unter einem leichten Überdruck steht, ist es notwendig, daß die Verbindung zwischen dem Hauptkörper und der Hülse gasdicht ist. Aus diesem Grund hat der Hauptkörper in einer vorteilhaften Weiterbildung der Erfindung eine ringförmige Anlagefläche, gegen die die Bördelung durch die Überwurfmutter abdichtend gedrückt ist. Die Überwurfmutter gewährleistet insbesondere gleichzeitig eine exakte axiale Ausrichtung mit der axialen Öffnung im Hauptkörper.

Zweckmäßigerweise umfaßt die Überwurfmutter die Hülse nahezu mit Gleitsitz, so daß im Hauptkörper eine gute Zentrierung und sichere Abdichtung erreicht wird.

Zur weiteren Verbesserung der Abdichtung kann zwischen dem proximalen Hülsenende und dem Hauptkörper ein Dichtungsring eingebracht werden. Dieser Dichtungsring liegt dann vorzugsweise zwischen der Anlagefläche des Hauptkörpers und der Bördelung der Hülse.

Wenn die Hülse als kreiszylindrisches Rohr mit distaler Abschrägung ausgebildet ist, ist der Perforationswiderstand der Trokarhülse beim Durchstoßen der Bauchdecke besonders gering. Die kreiszylindrische Gestaltung gestattet aber auch ein sicheres Zentrieren am Hauptkörper.

Aufgrund der raschen, manuellen Auswechselbarkeit der Hülse kann die Trokarhülse auch jeder Bauchdeckenstärke schnell angepaßt werden, so daß ein größerer Vorrat relativ teurer, kompletter, aber unterschiedlich langer Trokarhülsen vermieden werden kann. Zudem stehen durch die Erfindung nunmehr stets scharfe Hülsen bzw. Trokarspitzen zur Verfügung, so daß traumatische Effekte, wie sie vorausgehend angeführt wurden, paralysiert werden können. Ein Einschlüpfen der Hülse in den Körper ist nach menschlichem Ermessen einerseits aufgrund der proximalen Aufbördelung an der Hülse und andererseits erst recht durch die radiale Größe der Überwurfmutter nicht vorstellbar. Die Erfindung eröffnet daher im Hinblick auf Zuverlässigkeit der Trokarhülse, ihre Handhabbarkeit und auch ihrer Kosten eine gravierende Verbesserung gegenüber den in der Praxis bisher eingesetzten Trokarhülsen.

Vorteilhafte Weiterbildungen der Erfindung sind Gegenstand der Unteransprüche.

Die Erfindung wird nachfolgend beispielsweise anhand der schematischen Zeichnung beschrieben. In dieser zeigen:

Fig. 1 eine Seitenansicht einer Trokarhülse mit einer durch eine Überwurfmutter an einen Hauptkörper angeschraubten Hülse und

Fig. 2 einen längsgeschnittenen, vergrößerten Ausschnitt der Verbindungsstelle zwischen Hauptkörper und Hülse aus Fig. 1.

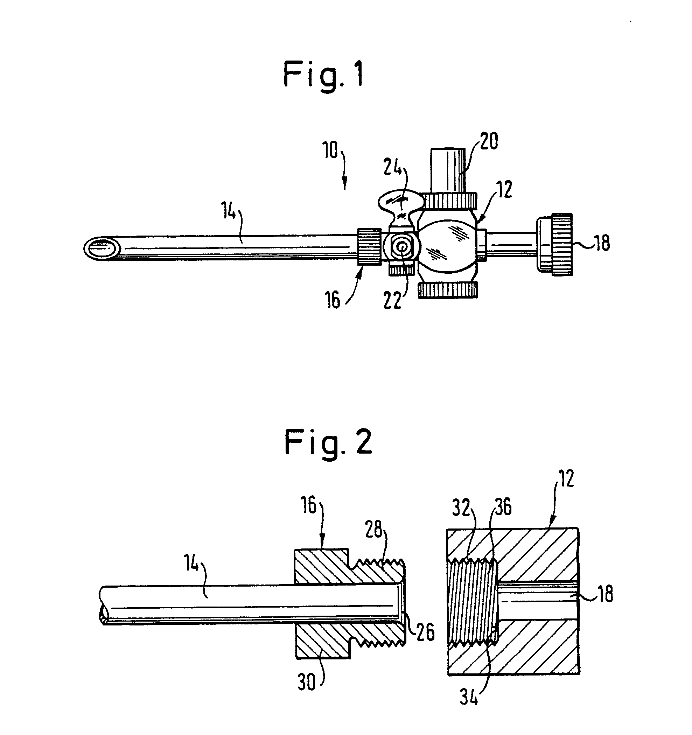

Die Trokarhülse 10 besteht aus einem Hauptkörper 12 und einer Hülse 14, die an ihrem proximalen Ende mittels einer Überwurfmutter 16 an dem Hauptkörper festgelegt ist. Der Hauptkörper 12 hat eine axiale Öffnung 18 (Fig. 2), deren Achse genau mit der Achse der Hülse 14 fluchtet.

Im Beispiel hat die Trokarhülse 10 des weiteren ein in Schließstellung vorgespanntes Ventil, das durch Druck auf den Ventilschaft 20 geöffnet werden kann, um durch die axiale Öffnung 18 chirurgische Instrumente hindurchführen zu können.

Fig. 2 zeigt die Verbindung zwischen Hülse 14 und Hauptkörper 12 im Detail. Die Hülse 14 hat an ihrem proximalen Ende eine Bördelung 26, die von der Überwurfmutter 16 hintergriffen wird. Die Überwurfmutter 16 ist in axialer Richtung in zwei Bereiche unterteilt. Der proximale Teil 28 ist als Anschlußbereich mit einem Außengewinde ausgebildet. Der distale Teil 30 der Überwurfmutter 16 ist als Griffbereich ausgebildet und an seinem kreiszylindrischen Außenumfang mit einer Rändelung versehen, um das Einschrauben des Außengewindes 28 in ein an dem Hauptkörper 12 ausgebildetes Innengewinde 32 zu erleichtern. Im eingeschraubten Zustand der Überwurfmutter 16 in den Hauptkörper 12 ist die Bördelung 26 durch die Überwurfmutter 16 an eine ringförmige radiale Anlagefläche 34 angedrückt, die im Hauptkörper am Ende eines erweiterten Verschraubungsbereichs 36 mit dem Innengewinde 32 angeordnet ist. Auf diese Weise wird eine gute Dichtigkeit an der Verbindungsstelle zwischen Hülse 14 und Hauptkörper 12 erreicht.

Die Hülse 14 und die Überwurfmutter 16 sind kreiszylindrisch ausgebildet, und die Überwurfmutter 16 umfaßt die Hülse 14 mit Gleitsitz. Auf diese Weise erhält man eine gute Zentrierung der Hülse 14 an dem Hauptkörper 12, so daß die axiale Öffnung 18 in dem Hauptkörper 12 und die Hülse 14 genau fluchten. Dies ist wichtig, damit an der Verbindungsstelle zwischen Hülse 14 und Hauptkörper 12 keine Kante ausgebildet ist, an der chirurgische oder optische Instrumente hängenbleiben oder beschädigt werden können.

Durch die erfindungsgemäße Verbindung von Hülse 14 und Hauptkörper 12 wird die Funktionssicherheit und die Handhabung der Trokarhülse deutlich verbessert. Der apparative Aufwand für chirurgische Instrumente kann zudem verringert werden, da nicht für jede Hülsenlänge und für jeden Hülsendurchmesser eine komplette Trokarhülse vorgesehen werden muß.

**Ansprüche**

1. Trokarhülse
mit einem proximalen Hauptkörper mit axialer durchgehender Öffnung, und mit einer mit dem Hauptkörper verbundenen distalen Hülse,
dadurch **gekennzeichnet,**
daß die Hülse (14) lösbar, insbesondere manuell auswechselbar mit dem Hauptkörper (12) verbunden ist.

2. Trokarhülse nach Anspruch 1,
dadurch **gekennzeichnet,**
daß die Hülse (14) an ihrem proximalen Ende aufgebördelt und von einer die Bördelung (26) hintergreifenden Überwurfmutter (16) umfaßt ist, die form- und/ oder kraftschlüssig an dem Hauptkörper (12) festgelegt ist.

3. Trokarhülse nach Anspruch 2,
dadurch **gekennzeichnet,**
daß die Überwurfmutter (16) ein Innen- oder Außengewinde (28) aufweist, das in ein dazu komplementäres an dem Hauptkörper (12) ausgebildetes Außen- oder Innengewinde (32) eingreift.

4. Trokarhülse nach Anspruch 2,
dadurch **gekennzeichnet,**
daß die Überwurfmutter mit dem Hauptkörper durch einen Bajonettverschluß verbunden ist.

5. Trokarhülse nach einem der Ansprüche 2 bis 4,
dadurch **gekennzeichnet,**
daß die Überwurfmutter (16) zwei axial hintereinander angeordnete Umfangsbereiche (28, 30) aufweist, von denen einer als Anschlußbereich (28) mit dem Hauptkörper (12) und der andere als Griffbereich (30) ausgebildet ist.

6. Trokarhülse nach Anspruch 5,
dadurch **gekennzeichnet,**
daß der Griffbereich (30) der Überwurfmutter (16) aufgerauht, insbesondere mit äußerer Rändelung versehen ist.

7. Trokarhülse nach einem der Ansprüche 2 bis 6,
dadurch **gekennzeichnet,**
daß der Hauptkörper (12) eine ringförmige Anlagefläche (34) aufweist, gegen die die Bördelung (26) durch die Überwurfmutter (16) abdichtend gedrückt ist.

8. Trokarhülse nach einem der Ansprüche 2 bis 7,
dadurch **gekennzeichnet,**
daß die Überwurfmutter (16) die Hülse (14) nahezu mit Gleitsitz umfaßt.

9. Trokarhülse nach einem der Ansprüche 2 bis 8,
dadurch **gekennzeichnet,**
daß die Hülse (14) mittels der Überwurfmutter (16) axial mit der axialen Öffnung (18) im Hauptkörper (12) ausgerichtet ist.

10. Trokarhülse nach einem der vorhergehenden Ansprüche,
dadurch **gekennzeichnet,**
daß zwischen dem proximalen Hülsenende und dem Hauptkörper ein Dichtungsring einbringbar ist.

11. Trokarhülse nach einem der vorhergehenden Ansprüche,
dadurch **gekennzeichnet,**
daß die Hülse (14) als kreiszylindrisches Rohr mit distaler Abschrägung ausgebildet ist.

# Fig. 1

# Fig. 2